# EUROPEAN PATENT SPECIFICATION

(11) **EP 3 773 428 B1**
(45) Date of publication and mention of the grant of the patent: **24.11.2021**
(21) Application number: 19706295.3
(22) Date of filing: 28.02.2019
(51) Int. Cl.: A61K 8/26, A61K 8/20, A61K 8/44, A61Q 15/00, B65D 83/00, A61K 8/04

(54) **ANTIPERSPIRANT PRODUCTS**
SCHWEISSHEMMENDE PRODUKTE
PRODUITS ANTI-TRANSPIRANTS

(30) Priority: 29.03.2018 EP 18164854
(43) Date of publication of application: 17.02.2021
(73) Proprietor: Unilever IP Holdings B.V., 3013 AL Rotterdam (NL); Unilever Global IP Limited, Wirral, CH62 AZD (GB)
(72) Inventor: ASKEM, Harriet, Jade, Leeds, Yorkshire LS14 2AR (GB); LUCKWELL, Craig, James, Leeds, Yorkshire LS14 2AR (GB)
(74) Representative: Whaley, Christopher
(86) International application number: PCT/EP2019/055000
(87) International publication number: WO 2019/185284

(56) References cited:
- WO-A1-01/27351
- WO-A1-2006/050776
- WO-A1-2016/066528
- WO-A1-2017/076836
- US-A- 6 136 303

## Description

### Field of Invention

The present invention is in the field of cosmetic antiperspirant compositions and dispensers therefor; in particular, aerosol compositions and dispensers therefor.

### Background

Antiperspirant (AP) aerosol compositions have been widely described and marketed for many years. Dispensers for such compositions have been disclosed in the prior art, as have AP compositions comprising aluminium sesquichlorohydrate activated by a water-soluble calcium salt and an amino acid.

WO 2016/066528 (Unilever, 2016) discloses anhydrous AP aerosol compositions comprising an AP active which is an aluminium sesquichlorohydrate activated by a water-soluble calcium salt and an amino acid.

WO 2017/076836 (Unilever, 2017) discloses an anhydrous AP aerosol compositions comprising an AP active which is an aluminium sesquichlorohydrate activated by a water-soluble calcium salt, which is contained within and dispensed from an aerosol dispenser having an aerosol valve having a vapor phase tap (VTP) to restrictive tailpiece (RTP) ratio of from 0.6:1 to 1.2:1.

WO 2006/050776 (Unilever, 2006) discloses an AP aerosol product comprising a water-in-oil emulsion AP composition in an aerosol dispenser having an aluminium can body internally lacquered with polyamide imide resin and mounting cup lacquered on its bottom side with epoxyphenolic vinyl lacquer.

### Summary of Invention

A problem associated with AP aerosol compositions is that the acidic nature of the AP active can lead to corrosion of various components of the aerosol dispenser. This is a particular problem with AP aerosol compositions comprising water, but it can also be an issue for anhydrous compositions. AP aerosol compositions are particularly susceptible to corrosion when the AP active is particularly acidic; for example, when the AP active comprises aluminium sesquichlorohydrate (ASCH).

Corrosive damage to the aerosol dispenser is most severe on the metal components, notably the internal surfaces of the can and the valve cup (otherwise known as the mounting cup). When a metal spring is included in the valve, this can also be particularly susceptible to corrosive damage.

We have also discovered that corrosive damage can occur to certain non-metal components of antiperspirant aerosol dispensers and the present invention is also concerned with alleviating this problem.

Herein, the aerosol dispenser and components thereof are to be understood to be "hardware".

It is an object of the present invention to provide an antiperspirant aerosol product having a reduced susceptibility to corrosive damage.

It is a further object of the present invention to provide an antiperspirant aerosol product comprising ASCH that has a reduced susceptibility to corrosive damage.

We have found that certain AP aerosol compositions comprising ASCH deliver surprisingly low levels of corrosion on aerosol hardware, particularly with selected hardware components as detailed herein.

In a first aspect of the invention, there is provided an antiperspirant aerosol product comprising:
A) an antiperspirant (AP) aerosol composition comprising a volatile propellant and an AP active comprising a basic aluminium chloride compound of formula Al₂OH_{4.4}Cl_{1.6} to Al₂OH_{4.9}Cl_{1.1}, a water-soluble calcium salt and an amino acid, and
B) an aerosol dispenser comprising a container body comprising a chamber for holding the aerosol composition and an aerosol valve through which the composition may be released through a valve stem of the aerosol valve, the aerosol valve comprising a housing holding the valve stem and a spring, said aerosol valve being held into the container body by a valve cup internally lacquered with a protective coating,
   wherein the AP active has a molar ratio of calcium to aluminium of greater than 1:20 and a molar ratio of glycine to aluminium of greater than 1:5.

In a second aspect of the invention, there is provided a method of achieving an antiperspirancy benefit on the human skin comprising the use an antiperspirant aerosol product according to the first aspect of the invention.

The second aspect of the invention referred to above may alternatively be expressed as the topical application to the skin of the human body of:
(A) an anhydrous AP aerosol composition comprising a volatile propellant and an AP active comprising a basic aluminium chloride compound of formula Al₂OH_{4.4}Cl_{1.6} to Al₂OH_{4.9}Cl_{1.1}, a water-soluble calcium salt and an amino acid, using
(B) an aerosol dispenser comprising a container body comprising a chamber for holding the aerosol composition and an aerosol valve through which the composition may be released through a valve stem of the aerosol valve, the aerosol valve comprising a housing holding the valve stem and a spring, said aerosol valve being held into the container body by a valve cup internally lacquered with a protective coating, wherein the AP active has a molar ratio of calcium to aluminium of greater than 1:20 and a molar ratio of glycine to aluminium of greater than 1:5.

In a third aspect of the invention, there is provided a method of manufacture of an antiperspirant aerosol product according to the first aspect of the invention.

In a fourth aspect of the invention, there is provided a method of reducing corrosive damage to an aerosol valve and/or an associated valve cup of an aerosol dispenser, the aerosol valve comprising a housing holding the valve stem and a spring, and being held in a container body by the valve cup, the corrosive damage being caused by an anhydrous AP aerosol composition comprising a volatile propellant and an AP active comprising a basic aluminium chloride compound of formula Al₂OH_{4.4}Cl_{1.6} to Al₂OH_{4.9}Cl_{1.1}, and the reduction in corrosive damage being brought about by the use of a protective coating on the internal surface of the valve cup and the addition of a water soluble calcium salt and an amino acid to the AP active such that the resulting AP active has a molar ratio of calcium to aluminium of greater than 1: 20 and a molar ratio of glycine to aluminium of greater than 1: 5.

### Detailed Description

Herein, all amounts, percentages, parts and ratios are by weight, unless otherwise indicated.

Herein, all features described as "preferred" are to be understood as particularly preferred when used in combination with any other feature described as "preferred".

Herein, features expressed as "preferred" with regard to a particular aspect of the invention should be understood to be preferred with regard to each aspect of the invention (likewise, features expressed as "more preferred" or "most preferred").

Herein, the word "comprising" is intended to mean "including" but not necessarily "consisting of", i.e., it is non-exhaustive.

Herein, locational terms, such as terms denoting relative positioning, such as "lower", "top", "bottom", refer to the aerosol dispenser in its upright orientation, i.e. with the valve towards top.

The AP composition comprises an AP active, a volatile propellant and may comprise other optional components.

Herein, the "base" of an aerosol composition is all the components of the composition *minus* the volatile propellant.

The base: propellant ratio of compositions used in accordance with the present invention can vary widely. The base to propellant ratio is preferably from 5: 95 to 50: 50, more preferably from 5: 95 to 40: 60 and most preferably from 10: 90 to 30: 70.

The present invention encompasses both "Regular" AP aerosol compositions and "Compressed" AP aerosol compositions. Regular AP aerosol compositions have a base to propellant ratio that preferably ranges from 5: 95 to less than 19: 81 and more preferably from 10: 90 to 16: 84. Compressed AP aerosol compositions have a base to propellant ratio that preferably ranges from 19: 81 to 40: 60 and more preferably from 19: 81 to 30: 70.

The AP aerosol composition and the base from which it is prepared are preferably water-in-oil emulsions or an anhydrous compositions.

The AP aerosol composition and the base from which it is preferred are more preferably anhydrous compositions.

AP aerosol compositions that are anhydrous are typically anhydrous suspensions of the AP active in a carrier fluid.

Herein, an "anhydrous" composition is one having less than 1 % by weight of free water and preferably less than 0.1% by weight of free water.

Herein, "free water" excludes any water of hydration associated with the antiperspirant salt or other component, but includes all other water present.

The present invention involves the use of an AP active comprising a basic aluminium chloride compound of formula Al₂OH_{4.4}Cl_{1.6} to Al₂OH_{4.9}Cl_{1.1}, a water-soluble calcium salt and an amino acid.

Herein, the basic aluminium chloride compound of formula Al₂OH_{4.4}Cl_{1.6} to Al₂OH_{4.9}Cl_{1.1} is aluminium sesquichlorohydrate (ASCH). The ASCH preferably has the formula Al₂OH_{4.7}Cl_{1.3} to Al₂OH_{4.9}Cl_{1.1}.

The ASCH is used in combination with a water-soluble calcium salt and an amino acid. These additional components serve to enhance the antiperspirancy efficacy of the ASCH, as described in WO 2017/076836 (Unilever, 2017) referred to earlier.

The water-soluble calcium salt is preferably calcium chloride and amino acid is preferably glycine.

A key feature of the present invention is that by carefully selecting the molar ratios of calcium to aluminium and, in particular, the glycine to aluminium in the AP active, corrosion to the aerosol dispenser can be resisted. The corrosion resistance may be delivered to one or more of the components of the aerosol dispenser.

The invention requires that the molar ratio of calcium to aluminium is greater than 1: 20 and that the molar ratio of glycine to aluminium is greater than 1: 5. It is preferred that the AP active has a molar ratio of glycine to aluminium of greater than 1: 4 and more preferably greater than 1: 3.8. At these preferred and more preferred ratios, the anti-corrosion benefits of the invention are particularly visible.

Herein, references to molar amounts and ratios of "aluminium" are calculated on the basis of mononuclear aluminium, but include aluminium present in poly-nuclear species; indeed, most of the aluminium in the salts of relevance is present in poly-nuclear species.

The presence of water-soluble calcium salt and amino acid may also serve to "activate" the ASCH, i.e., to enhance its antiperspirancy efficacy. This enhancement may be brought about by heating the ASCH with the calcium salt and amino acid prior to formulation, as described in WO 2014/187685 (Unilever, 2014).

The ASCH, water-soluble calcium salt and amino acid may be co-spray dried to give a particulate powder. "Co-spray drying" means that a homogeneous solution of the components is spray-dried. The resulting particulates may optionally be milled to give a desired particle size before incorporation into an anhydrous aerosol suspension composition.

When used in particulate form, the AP active preferably has a mean particle size (D50) of at least 6 microns, more preferably at least 17 microns, and most preferably from 20 to 30 microns.

Herein, mean (D50) particle sizes may be measured using (laser) light scattering techniques, for example using a Mastersizer instrument, obtainable from Malvem Instruments. Such instruments are set to produce a volume plot and a lens is selected in accordance with the maker's instructions to accommodate the expected particle size distribution, (or various lenses can be tested until the best lens is identified). Measurements are made by methods known in the art.

The particulate antiperspirant active is preferably spray-dried using rotary atomisation.

A volatile propellant is an essential component of compositions of the invention. Preferred volatile propellants are liquefied propellant gases, in particular hydrocarbons or halogenated hydrocarbon gases (particularly fluorinated hydrocarbons such as 1,1-difluoroethane and/or 1-trifluoro-2-fluoroethane) that have a boiling point of below 10°C and especially those with a boiling point below 0°C. It is especially preferred to employ liquefied hydrocarbon gases, and especially C₃ to C₆ hydrocarbons, including propane, butane, isobutane, pentane and isopentane and mixtures of two or more thereof. Of these especially preferred propellants, isobutane, isobutane/propane, butane/propane and mixtures of propane, isobutane and butane are most preferred.

The liquefied propellant gas is typically the major component of aerosol compositions, often comprising from 30 to 99% weight and preferably comprising from 50 to 95% by weight.

A highly preferred component is a carrier fluid, typically a carrier oil, for the AP active. In preferred embodiments, this may also be a masking oil, serving the purpose of reducing visible deposits when the composition accidentally meets clothing, for example.

Herein, the terms "oil" and signifies a water-insoluble organic material that is liquid at 20°C. Any material having a solubility of less than 0.1g/100g at 20°C is considered to be insoluble.

A preferred optional component for use in accordance with the present invention is a fragrance oil, sometimes alternatively called a perfume oil. The fragrance oil may comprise a single fragrance or component more commonly a plurality of fragrance components. Herein, fragrance oils impart an odour, preferably a pleasant odour, to the composition. Preferably, the fragrance oil imparts a pleasant odour to the surface of the human body the composition is applied to the same.

The amount of fragrance oil in the composition is commonly up to 3% advantageously is at least 0.5% and particularly from 0.8% to 2%.

The total amount of carrier oil in the composition is preferably from 0.1 to 20%, more preferably from 0.5 to 10%, and most preferably at from 2 to 8% by weight of the total composition. In certain preferred embodiments, the carrier oil is present at greater than 2.5% and less than 6% by weight of the total composition.

The carrier oil may be selected from any of those known in the art, although hydrophobic carrier oils are preferred.

A preferred class of carrier oil are silicone oils, i.e., liquid polyorganosiloxanes. Such materials may be cyclic or linear, examples include Dow Corning silicone fluids 344, 345, 244, 245, 246, 556, and the 200 series; Union Carbide Corporation Silicones 7207 and 7158; and General Electric silicone SF1202.

Suitable carrier oils can be selected from alkyl ether oils having a boiling point of above 100°C and especially above 150°C, including polyalkyleneglycol alkyl ethers. Such ethers desirably comprise between 10 and 20 ethylene glycol or propylene glycol units and the alkyl group commonly contains from 4 to 20 carbon atoms. The preferred ether oils include polypropylene glycol alkyl ethers such as PPG-14-butylether and PPG-15-stearyl ether.

Suitable carrier oils can include one or more triglyceride oils. The triglyceride oils commonly comprise the alkyl residues of aliphatic C₇ to C₂₀ alcohols, the total number of carbon atoms being selected in conjunction with the extent of olefinic unsaturation and/or branching to enable the triglyceride to be liquid at 20°C. One example is jojoba oil. Particularly preferably, in the triglyceride oil the alkyl residues are linear C₁₈ groups having one, two or three olefinic degrees of unsaturation, two or three being optionally conjugated, many of which are extractable from plants (or their synthetic analogues), including triglycerides of oleic acid, linoleic acid, conjugated linoleic acids, linolenic acid, petroselenic acid, ricinoleic acid, linolenelaidic acid, trans 7-octadecenoic acid, parinaric acid, pinolenic acid, punicic acid, petroselenic acid and stearidonic acid.

Suitable carrier oils can include those derived from unsaturated C₁₈ acids, including coriander seed oil, impatiens balsimina seed oil, parinarium laurinarium kernel fat oil, sabastiana brasilinensis seed oil, dehydrated castor seed oil, borage seed oil, evening primrose oil, aquilegia vulgaris oil, sunflower (seed) oil and safflower oil. Other suitable oils are obtainable from hemp, and maize corn oil. An especially preferred oil by virtue of its characteristics is sunflower (seed) oil.

Further suitable carrier oils, that can also be emollient oils, comprise alkyl or alkyl-aryl ester oils having a boiling point of above 150°C (and a melting point of below 20°C). Such ester oils include oils containing one or two alkyl groups of 12 to 24 carbon atoms length, including isopropyl myristate, isopropyl palmitate and myristyl palmitate. Other non-volatile ester oils include alkyl or aryl benzoates such C₁₂₋₁₅ alkyl benzoate, for example Finsolv TN™ or Finsolv Sun™.

A further class of suitable carrier oils comprises non-volatile dimethicones, often comprising phenyl or diphenylene substitution, for example Dow Corning 200 350cps or Dow Corning 556.

A suspending agent is a highly preferred component of compositions of the invention. Such agents aid the suspension of particulate AP active in the composition. Preferred suspending agents are clays, particularly hydrophobically modified clays. Particularly preferred are hydrophobically modified hectorite or bentonite clays and especially preferred is disteardimonium hectorite (e.g. Bentone 38V, ex Elementis).

The suspending agent is typically employed at from 0.1 to 1.5% by weight of the total composition.

Propylene carbonate may also be advantageously employed in compositions of the present invention, typically at from 0.001 to 0.1% by weight.

The aerosol dispenser comprises a container body comprising a chamber for holding the aerosol composition and an aerosol valve through which the composition may be released through an aerosol valve.

The container body forms the bulk of the aerosol dispenser and comprises a chamber for holding the aerosol composition.

The container body may be made of tin-plated steel or aluminium, but is preferably made of aluminium.

In preferred embodiments, the container body has no seams, i.e. it is a one-piece container, typically prepared by impact extrusion. This is particularly true for aluminium container bodies.

The internal surface of the container body/chamber is typically lacquered to protect it from corrosion. Suitable lacquers include epoxyphenolic resin (also called epon-phenolic and often abbreviated to "epoxy" or "EPON"); poly(ethylene terephthalate) (PET); polypropylene; EP-vinyl lacquer (also called organosol or Micoflex); and polyamide imide resin (also called PAM or PAI).

The aerosol valve is a standard component of aerosol dispensers and is essential for allowing the composition to exit the dispenser. In use, opening of the valve allows the antiperspirant aerosol composition to leave the container body via a nozzle and thereby produce a spray.

The aerosol valve comprises a valve stem, a valve stem housing and a valve spring. The valve stem serves to allow passage of the composition from the container when actuated, typically by depression. The valve stem housing serves to hold the valve stem in it places within a hole in the centre of the valve cup (see below). The valve spring serves to restore the valve stem to its original position following actuation.

The valve stem and valve stem housing are both typically made of plastic; however, both may be damaged by the antiperspirant composition contained with the dispenser. Suitable plastics for the valve stem are polyamides, such as nylon, polyolefins, such as polypropylene or polyethylene, or acetal. Suitable plastics for the valve stem housing are polyamides, such as nylon, and acetal.

For use in accordance with the present invention, our studies have revealed that the valve stem is preferably made from a polyamide, such as nylon, or a polyolefin, such as polypropylene or polyethylene. A polyamide, such as nylon, is particularly preferred and nylon-66 is especially preferred.

For use in accordance with the present invention, our studies have revealed that the valve stem housing is preferably made from a polyamide, such as nylon, and especially nylon-66.

It is particularly preferred to have both the valve stem and the valve stem housing made from nylon and especially nylon-66.

The valve spring is typically made of stainless steel.

The valve cup is a standard component of aerosol dispensers and is otherwise known as the mounting cup. The valve cup may be made of tin-plated steel or aluminium, but is preferably made of tin-plated steel.

The valve cup serves to close off the container body at its top, typically at a narrowed or 'necked' section of the body. The internal surface of the valve cup meets the composition contained within the chamber, particularly when the can is inverted. The valve cup has the valve present in a hole in its centre.

The valve cup may be lacquered on its external surface; however, it is its internal surface that is more frequently in contact with the composition and this side that must be treated with a protective coating.

The protective coating on the valve cup is a layer of material, known as a lacquer, applied to the surface of the valve cup by methods known in the art. The layer is preferably of thickness from 1 to 500 microns at all points on the internal surface of the can. The high maximum is due to the difficulty of applying lacquer to the valve cup because of its highly-contoured nature. The average thickness of the layer is preferably from 1 to 50 microns and may be measured by making 10 or more measurements at random positions on the bottom side of the valve cup.

A range of lacquers are commercially available and are potentially suitable for the treatment of the internal surface of the chamber and the internal (and optionally external) surface of the valve cup. Examples include epoxyphenolic resin (also called epon-phenolic and often abbreviated to "epoxy" or "EPON"); poly(ethylene terephthalate) (PET); polypropylene; EP-vinyl lacquer (also called organosol or Micoflex); and polyamide imide resin (also called PAM or PAI).

In preferred embodiments, the valve cup is internally lacquered with PET or an epoxyphenolic resin. It is particularly preferred that this resin is an epoxyphenolic resin and especially preferred that the selected resin has an average thickness of from 1 to 50 microns.

### Examples

A series of corrosion studies were performed using compositions prepared from the AP actives details in Table 1. These AP actives were prepared by heating together an aqueous solution of the components indicated in Table 1 for 2 hours at 87°C, the concentration of the aqueous solution being 27% total solids (+/-1%). The resulting solution was spray dried to give particulate AP active samples.

**Table 1**

| **Sample Ref.** | **Weight ratio of anhydrous ingredients** | | | **Molar ratio of anhydrous ingredients** | | |
|---|---|---|---|---|---|---|
| | ASCH | CaCl₂ | Glycine | Al in ASCH* | CaCl₂ | Glycine |
| **A** | 12.00 | 0.00 | 0.00 | 10.00 | 0.00 | 0.00 |
| **B** | 12.00 | 1.50 | 1.17 | 10.00 | 1.00 | 1.16 |
| **C** | 12.00 | 0.90 | 2.00 | 10.00 | 0.60 | 1.98 |
| **1** | 12.00 | 1.50 | 2.05 | 10.00 | 1.00 | 2.03 |
| **2** | 12.00 | 1.50 | 2.93 | 10.00 | 1.00 | 2.90 |
| **3** | 12.00 | 1.50 | 3.81 | 10.00 | 1.00 | 3.77 |
| **4** | 12.00 | 1.50 | 4.70 | 10.00 | 1.00 | 4.65 |

| | | | | | | |
|---|---|---|---|---|---|---|
| * ASCH has two moles of Al per mole ASCH, so 10 mole% Al in ASCH = 5 mole% ASCH. | | | | | | |

These AP actives were formulated into APA compositions as detailed in Table 2. The bases for the APA compositions were prepared by methods known in the art and were then gassed with propellant (propane, butane, isobutane) to give the "Regular" and "Compressed" APA compositions as indicated.

**Table 2**

| Component | **Regular** | | **Compressed** | |
|---|---|---|---|---|
| Cyclopentasiloxane | 5.60 | 7.60 | 10.75 | 14.25 |
| AP active | 3.00 | 7.00 | 5.63 | 13.13 |
| Fragrance | 0.80 | 0.80 | 1.50 | 1.50 |
| Disteardimonium hectorite | 0.50 | 0.50 | 0.94 | 0.94 |
| Propylene carbonate | 0.10 | 0.10 | 0.19 | 0.19 |
| Propellant | 90 | 84 | 81 | 70 |

### Corrosion Tests - Series 1

This test used the Compressed compositions as detailed in Table 2, prepared with each of the AP actives indicated in Table 3 and described in greater detail in Table 1. The compositions were filled into standard aluminium aerosol cans and sealed with a valve cup and valve. The filled cans were stored at 25°C and 45°C in both upright and inverted positions. The extent of corrosion of the internal surface of the valve cups was assessed after storage for 12 weeks. The valve cups were tin plate steel with an epoxyphenolic lacquer.

Corrosion scores were assigned on a 0 to 10 line-scale wherein:
0 = no corrosion
1 = a few, tiny corrosion marks
2 = many, tiny corrosion marks
3 = a few, small corrosion marks
4 = very many, tiny corrosion marks
5 = many, small corrosion marks
6 = a few, medium size corrosion marks
7 = very many, small corrosion marks
8 = a few, big corrosion marks
9 = many, big corrosion marks
10 = very many, big corrosion marks.

**Table 3**

| | | | **Corrosion Scores** | | | |
|---|---|---|---|---|---|---|
| | | | **B** | **C** | **A** | **4** |
| At 25°C | Upright | | | | | |
| | Active level: | 5.6% | 3 | 6 | 5 | 1 |
| | | 13.1% | 3 | 7 | 5 | 1 |
| | Inverted | | | | | |
| | Active level: | 5.6% | 1 | 5 | 3 | 1 |
| | | 13.1% | 5 | 3 | 3 | 0 |
| At 45°C | Upright | | | | | |
| | Active level: | 5.6% | 8 | 5 | 6 | 3 |
| | | 13.1% | 7 | 7 | 5 | 1 |
| | Inverted | | | | | |
| | Active level: | 5.6% | 4 | 4 | 5 | 3 |
| | | 13.1% | 7 | 7 | 5 | 1 |

From the results detailed in Table 3, several conclusions may be drawn, including:
- The samples stored at 45°C showed more corrosion that those stored at 25°C.
- The nature of the active in the composition had a major influence on the level of corrosion, with corrosion being significantly less for the composition used in accordance with the invention.

### Corrosion Tests - Series 2

In a further study, a series of accelerated corrosions tests were performed as described below. In these tests, aerosol valve cups were placed in jars and contacted with APA bases under conditions significantly more extreme than would be the case in normal usage of APA compositions in aerosol dispensers. The APA base compositions used are indicated in Table 4 and correspond to the APA bases used to prepare the APA compositions indicated in Table 2.

The valve cups were tin plated steel with an epoxyphenolic lacquer (on all surfaces). Valve cups from two independent suppliers were investigated, designated "Supplier 1" and "Supplier 2". The APA active used in each base composition is as indicated in Table 5, with further details in Table 1.

**Table 4**

| Component | **A** | **B** |
|---|---|---|
| Cyclopentasiloxane | 56 | 47.5 |
| AP active | 30 | 43.75 |
| Fragrance | 8 | 5.00 |
| Disteardimonium hectorite | 5 | 3.125 |
| Propylene carbonate | 1 | 0.625 |

In this study, corrosion of the valve cup was assessed using the following line-scale:
0 = no corrosion
1 = some corrosion on edges
2 = edges and stem top showing corrosion
3 = really bad corrosion on edges
4 = edges corroded and marks on bottom of valve cup
5 = edges corroded and marks on top and bottom of valve cup
6 = severe damage, black marks on all surfaces.

The results are shown in Table 5.

**Table 5**

| | | | **Corrosion Scores** | | | |
|---|---|---|---|---|---|---|
| | | | **B** | **C** | **A** | **4** |
| After 1 week | Supplier 1 | | | | | |
| | Active level: | 30% | 5 | 1 | 1 | 0 |
| | | 43.75% | 3 | 2 | 5 | 0 |
| | Supplier 2 | | | | | |
| | Active level: | 30% | 5 | 1 | 1 | 0 |
| | | 43.75% | 3 | 4 | 5 | 0 |
| After 2 weeks | Supplier 1 | | | | | |
| | Active level: | 30% | 5 | 2 | 4 | 1 |
| | | 43.75% | 5 | 4 | 5 | 0 |
| | Supplier 2 | | | | | |
| | Active level: | 30% | 5 | 2 | 4 | 1 |
| | | 43.75% | 5 | 2 | 5 | 0 |

This series of tests is illustrative of the reduced valve cup corrosion found using the method of the fourth aspect of the invention.

### Corrosion Tests - Series 3

An analogous series of corrosion tests to the one described above as "Series 2" was performed to assess the corrosion on valve springs from two different suppliers, designated "Supplier 1" and "Supplier 2", as before. In these tests, the valve springs were left in jars, half-covered with the base formulations, for one or two weeks at 45°C. The valve springs were made of stainless steel.

In this study, corrosion of the valve springs was assessed and a 0 to 5 line-scale was used to record the extent of corrosion, wherein:
0 = no corrosion and
5 = extensive corrosion.

The results are presented in Table 6.

**Table 6**

| | | | **Corrosion Scores** | | | |
|---|---|---|---|---|---|---|
| | | | **B** | **C** | **A** | **4** |
| After 1 week | Supplier 1 | | | | | |
| | Active level: | 30% | 5 | 3 | 4 | 1 |
| | | 43.75% | 5 | 4 | 5 | 2 |
| | Supplier 2 | | | | | |
| | Active level: | 30% | 5 | 3 | 4 | 1 |
| | | 43.75% | 5 | 4 | 5 | 2 |
| After 2 weeks | Supplier 1 | | | | | |
| | Active level: | 30% | 5 | 3 | 4 | 1 |
| | | 43.75% | 5 | 4 | 5 | 2 |
| | Supplier 2 | | | | | |
| | Active level: | 30% | 5 | 3 | 4 | 1 |
| | | 43.75% | 5 | 4 | 5 | 2 |

This series of tests is illustrative of the reduced valve spring corrosion found using the method of the fourth aspect of the invention.

### Corrosion Tests - Series 4

A further a series of accelerated corrosions tests was performed on following samples using the same method as described above for "Series 2". The assessment was made on valve cups having an epoxyphenolic lacquer, provided by two different suppliers, designated "Supplier 1" and "Supplier 2". Only one formulation was assessed (the APA base composition from Table 4 comprising 43.75% AP active), but this formulation was prepared using four different AP actives (B, 1, 2 and 3).

In this study, corrosion of the valve cup was assessed and the following corrosion indicators, increasing in severity, were employed:
0 = No corrosion.
1 = Very slight corrosion around the edges. Slight corrosion around the valve stem opening.
2 = Edges and around the valve stem opening showing some corrosion.
3 = Badly corroded edges. Slight corrosion around the valve stem opening and one corrosion spot on the top surface.
4 = Badly corroded edges and area around valve stem opening. Corrosive pitting on top and bottom surfaces.
5 = As #4, plus two corrosion spots on bottom surface.
6 = Bad corrosion on all areas around the valve stem opening, particularly on the edges.
7 = As #6, plus small black marks all over lower surface of valve cup.

The results are presented in Table 7.

**Table 7**

| Duration of test | Supplier | AP level | **Corrosion Scores** | | | |
|---|---|---|---|---|---|---|
| | | | **B** | **1** | **2** | **3** |
| After 1 week | Supplier 1 | 43.75% | 6 | 4 | 3 | 2 |
| | Supplier 2 | 43.75% | 6 | 4 | 3 | 1 |
| After 2 weeks | Supplier 1 | 43.75% | 7 | 5 | 3 | 2 |
| | Supplier 2 | 43.75% | 7 | 5 | 3 | 1 |

This series of tests is illustrative of the reduced valve cup corrosion found using the method of the fourth aspect of the invention for samples, the level of corrosion reducing according to AP active in the order: B > 1 > 2 > 3.

### Corrosion Tests - Series 5

A further series of accelerated corrosions tests was performed to assess the corrosive effects of various compositions upon valve cups having a different lacquer: polyethylene terephthalate (PET), as detailed below. As for the Series 4 tests, only a single base composition type was tested (the APA base composition from Table 4 comprising 43.75% AP active), this time formulated with three different AP actives: B, C and 4.

In this study, corrosion of the valve cup was assessed and a 0 to 7 line-scale was used to record the extent of corrosion, wherein:
0 = no corrosion
1 = slight corrosion and
7 = extensive corrosion.

In Table 8, the results of this study are contrasted with those from the Series 2 studies as detailed above, which employed a valve cup coated with epoxyphenolic resin (EPR).

**Table 8**

| | | | **Corrosion Scores** | | |
|---|---|---|---|---|---|
| | Valve resin | Active level | **B** | **C** | **4** |
| After 1 week | PET | 43.75% | 3 | 2 | 1 |
| | EPR | 43.75% | 3 | 2 | 0 |
| After 2 weeks | PET | 43.75% | 3 | 2 | 1 |
| | EFR | 43.75% | 5 | 4 | 0 |
| After 4 weeks | PET | 43.75% | 3 | 2 | 1 |
| | EPR | 43.75% | n/a | n/a | n/a |

| | | | | | |
|---|---|---|---|---|---|
| n/a = not available and indicates that this study was not performed. | | | | | |

These results are illustrative of the benefit of the fourth aspect of the present invention across two types of protective resin on the valve cup and of the advantage of an EFR protective resin on the valve cup, for ASCH-based AP active according to the present invention, under accelerated corrosion conditions. For the AP active 4 formulation, the EPR resin appeared to give reduced corrosion.

### Corrosion Tests - Series 6

A further series of corrosions tests was performed to assess the corrosive effects of various compositions upon valve stems and housings from a variety of suppliers. This study used a fully formulated Regular APA composition and was performed in accordance with the Series 1 corrosion tests. The aerosol products were stored in an upright orientation for 26 weeks at 45°C.

Further details and results are given in Table 9. The damage scores for both the housing and the stem material are indicated in a simple, binary manner:
0 = no damage,
1 = some damage.

The "some damage" indicator covered various types of damage; in particular, whitening or degradation of castellations on the housing and surface degradation of the stem.

The above results indicate that no damage was observed with housings made from polypropylene, polyethylene or nylon. The acetal housings, by contrast, suffered damage with both formulations tested.

Damage to the valve stem was limited and was only observed for acetal stems and a single nylon valve stem.

**Table 9**

| **Ref.** | | | | **Corrosion Scores** | | | |
|---|---|---|---|---|---|---|---|
| | **Valve supplier** | **Housing** | **Stem** | **C** | | **3** | |
| | | | | **Housing** | **Stem** | **Housing** | **Stem** |
| **T1** | 1 | Nylon | Nylon | 0 | 0 | 0 | 0 |
| **T2** | 1 | PP* | Nylon | 0 | 0 | 0 | 0 |
| **T3** | 1 | PE** | Nylon | 0 | 0 | 0 | 0 |
| **T4** | 1 | Acetal | Nylon | 1 | 0 | 1 | 0 |
| **T5** | 2 | Acetal | Acetal | 1 | 1 | 1 | 1 |
| **T6** | 3 | Nylon | Acetal | 0 | 0 | 0 | 0 |
| **T7** | 4 | Nylon | Acetal | 0 | 0 | 0 | 0 |
| **T8** | 5 | Nylon | Nylon | 0 | 1 | 0 | 1 |
| **T9** | 6 | Nylon | Nylon | 0 | 0 | 0 | 0 |
| **T10** | 2 | PE** | Acetal | 0 | 1 | 0 | 1 |

| | | | | | | | |
|---|---|---|---|---|---|---|---|
| * PP = polypropylene. ** PE = polyethylene. | | | | | | | |

## Claims

1. An antiperspirant aerosol product comprising:
A) an antiperspirant (AP) aerosol composition comprising a volatile propellant and an AP active comprising a basic aluminium chloride compound of formula Al₂OH_{4.4}Cl_{1.6} to Al₂OH_{4.9}Cl_{1.1}, calcium chloride and glycine, and
B) an aerosol dispenser comprising a container body comprising a chamber for holding the aerosol composition and an aerosol valve through which the composition may be released through a valve stem of the aerosol valve, the aerosol valve comprising a housing holding the valve stem and a spring, said aerosol valve being held into the container body by a valve cup internally lacquered with a protective coating,
wherein the AP active has a molar ratio of calcium to aluminium of greater than 1:20 and a molar ratio of glycine to aluminium of greater than 1:5.

2. An antiperspirant aerosol product according to claim 1, wherein the AP active has a molar ratio of glycine to aluminium of greater than 1:4 and preferably greater than 1: 3.8.

3. An antiperspirant aerosol product according to claim 1 or claim 2, wherein the valve cup and/or the internal surface of the chamber of the aerosol dispenser is internally lacquered with polyethylene terephthalate or an epoxyphenolic resin.

4. An antiperspirant aerosol product according to claim 3, wherein the valve cup and/or the internal surface of the chamber is internally lacquered with an epoxyphenolic resin.

5. An antiperspirant aerosol product according to any of preceding claims, wherein the housing for the aerosol valve of the aerosol dispenser is made of nylon or a polyolefin.

6. An antiperspirant aerosol product according to any of the preceding claims, wherein the valve cup and/or the chamber of the aerosol dispenser is made from tin-plated steel.

7. An antiperspirant aerosol product according to any of the preceding claims, wherein the valve spring of the aerosol dispenser is made from stainless steel.

8. An antiperspirant aerosol product according to any of the preceding claims, wherein the valve stem of the aerosol valve is made from nylon.

9. An antiperspirant aerosol product according to any of the preceding claims wherein the antiperspirant (AP) aerosol composition is anhydrous.

10. An antiperspirant aerosol product according to claim 10, wherein in the composition is suspension of AP active in a carrier fluid.

11. An antiperspirant aerosol product according to claim 10, wherein the composition comprises a suspending agent.

12. A method of achieving an antiperspirancy benefit on the human skin comprising the use an antiperspirant aerosol product according to any of the preceding claims.

13. A method of reducing corrosive damage to an aerosol valve and an associated valve cup of an aerosol dispenser, the aerosol valve comprising a housing holding the valve stem and a spring, and being held in a container body by the valve cup, the corrosive damage being caused by an AP aerosol composition comprising a volatile propellant and an AP active comprising a basic aluminium chloride compound of formula Al₂OH_{4.4}Cl_{1.6} to Al₂OH_{4.9}Cl_{1.1}, and the reduction in corrosive damage being brought about by the use of a protective coating on the internal surface of the valve cup and the addition of calcium chloride and glycine to the AP active such that the resulting AP active has a molar ratio of calcium to aluminium of greater than 1: 20 and a molar ratio of glycine to aluminium of greater than 1: 5.

## Patentansprüche

1. Antitranspirant-Aerosolprodukt, das Folgendes umfasst:
A) eine Antitranspirant-Aerosolzusammensetzung (AT-Aerosolzusammensetzung), die ein flüchtiges Treibmittel und einen AT-Wirkstoff umfasst, der eine Aluminiumchlorid-Basisverbindung der Formel Al₂OH_{4,4}Cl_{1,6} bis Al₂OH_{4,9}Cl_{1,1}, Calciumchlorid und Glycin enthält, und
B) einen Aerosolspender, der einen Behälterkörper umfasst, der eine Kammer zum Aufnehmen der Aerosol-Zusammensetzung und ein Aerosolventil, durch das die Zusammensetzung durch einen Ventilschaft des Aerosolventils ausgegeben werden kann, umfasst, wobei das Aerosolventil ein Gehäuse umfasst, das den Ventilschaft und eine Feder enthält, wobei das Aerosolventil im Behälterkörper durch einen Ventilbecher gehalten wird, der innen mit einer Schutzschicht lackiert ist,
wobei der AT-Wirkstoff ein Molverhältnis von Calcium zu Aluminium, das größer als 1:20 ist, und ein Molverhältnis von Glycin zu Aluminium, das größer als 1:5 ist, aufweist.

2. Antitranspirant-Aerosolprodukt nach Anspruch 1, wobei der AT-Wirkstoff ein Molverhältnis von Glycin zu Aluminium hat, das größer als 1:4 und vorzugsweise größer als 1:3,8 ist.

3. Antitranspirant-Aerosolprodukt nach Anspruch 1 oder Anspruch 2, wobei der Ventilbecher und/oder die innere Oberfläche der Kammer des Aerosolspenders innen mit Polyethylen-Terephtalat oder einem Epoxid-Phenolharz lackiert sind.

4. Antitranspirant-Aerosolprodukt nach Anspruch 3, wobei der Ventilbecher und/oder die innere Oberfläche der Kammer innen mit einem Epoxid-Phenolharz lackiert sind.

5. Antitranspirant-Aerosolprodukt nach einem der vorhergehenden Ansprüche, wobei das Gehäuse für das Aerosolventil des Aerosolspenders aus Nylon oder einem Polyolefin gefertigt ist.

6. Antitranspirant-Aerosolprodukt nach einem der vorhergehenden Ansprüche, wobei der Ventilbecher und/oder die Kammer des Aerosolspenders aus einem verzinnten Stahl gefertigt sind.

7. Antitranspirant-Aerosolprodukt nach einem der vorhergehenden Ansprüche, wobei die Ventilfeder des Aerosolspenders aus Edelstahl gefertigt ist.

8. Antitranspirant-Aerosolprodukt nach einem der vorhergehenden Ansprüche, wobei der Ventilschaft des Aerosolventils aus Nylon gefertigt ist.

9. Antitranspirant-Aerosolprodukt nach einem der vorhergehenden Ansprüche, wobei die Antitranspirant-Aerosolzusammensetzung (AT-Aerosolzusammensetzung) wasserfrei ist.

10. Antitranspirant-Aerosolprodukt nach Anspruch 10, wobei die Zusammensetzung eine Suspension eines AT-Wirkstoffs in einem Trägerfluid ist.

11. Antitranspirant-Aerosolprodukt nach Anspruch 10, wobei die Zusammensetzung ein Suspensionsmittel enthält.

12. Verfahren zum Erreichen einer Antitranspirant-Wirkung auf der menschlichen Haut, das die Verwendung eines Antitranspirant-Aerosolprodukts nach einem der vorhergehenden Ansprüche umfasst.

13. Verfahren zum Verringern von Korrosionsschäden an einem Aerosolventil und einem zugehörigen Ventilbecher eines Aerosolspenders, wobei das Aerosolventil ein Gehäuse umfasst, das den Ventilschaft und eine Feder hält, und in einem Behälterkörper durch den Ventilbecher gehalten wird, wobei der Korrosionsschaden durch eine AT-Aerosolzusammensetzung verursacht wird, die ein flüchtiges Treibmittel und einen AT-Wirkstoff umfasst, der eine Aluminiumchlorid-Basisverbindung der Formel Al₂OH_{4,4}Cl_{1,6} bis Al₂OH_{4,9}Cl_{1,1} umfasst, und wobei die Verringerung des Korrosionsschadens durch die Verwendung einer Schutzschicht auf der inneren Oberfläche des Ventilbechers und den Zusatz von Calciumchlorid und Glycin zum AT-Wirkstoff erzielt wird, so dass der resultierende AT-Wirkstoff ein Molverhältnis von Calcium zu Aluminium, das größer als 1:20 ist, und ein Molverhältnis von Glycin zu Aluminium, das größer als 1:5 ist, aufweist.

## Revendications

1. Produit d'aérosol antiperspirant comprenant :
(A) une composition d'aérosol antiperspirant (AP) comprenant un propulseur volatile et un actif AP comprenant un composé de chlorure d'aluminium basique de formule Al₂OH_{4,4}Cl_{1,6} à Al₂OH_{4,9}CH_{1,1}, du chlorure de calcium et de la glycine, et
(B) un distributeur d'aérosol comprenant un corps de récipient ayant une chambre pour contenir la composition d'aérosol et une vanne d'aérosol à travers laquelle la composition peut être libérée par une tige de vanne de la vanne d'aérosol, la vanne d'aérosol comprenant un logement contenant la tige de vanne et un ressort, ladite vanne d'aérosol étant contenue dans le corps de récipient par une coupelle de vanne laquée intérieurement avec un revêtement protecteur,
dans lequel l'actif AP présente un rapport molaire de calcium à aluminium supérieur à 1 : 20 et un rapport molaire de glycine à aluminium supérieur à 1 : 5.

2. Produit d'aérosol antiperspirant selon la revendication 1, dans lequel l'actif AP présente un rapport molaire de glycine à aluminium supérieur à 1 : 4 et de préférence supérieur à 1 : 3,8.

3. Produit d'aérosol antiperspirant selon la revendication 1 ou revendication 2, dans lequel la coupelle de vanne et/ou la surface interne de la chambre du distributeur d'aérosol est laquée intérieurement avec du poly(éthylène téréphtalate) ou une résine époxy phénolique.

4. Produit d'aérosol antiperspirant selon la revendication 3, dans lequel la coupelle de vanne et/ou la surface interne de la chambre est laquée intérieurement avec une résine époxy phénolique.

5. Produit d'aérosol antiperspirant selon l'une quelconque des revendications précédentes, dans lequel le logement pour la vanne d'aérosol du distributeur d'aérosol est constitué de nylon ou d'une polyoléfine.

6. Produit d'aérosol antiperspirant selon l'une quelconque des revendications précédentes, dans lequel la coupelle de vanne et/ou la chambre du distributeur d'aérosol est constituée d'acier plaqué d'étain.

7. Produit d'aérosol antiperspirant selon l'une quelconque des revendications précédentes, dans lequel le ressort de vanne du distributeur d'aérosol est constitué d'acier inoxydable.

8. Produit d'aérosol antiperspirant selon l'une quelconque des revendications précédentes, dans lequel la tige de vanne de la vanne d'aérosol est constituée de nylon.

9. Produit d'aérosol antiperspirant selon l'une quelconque des revendications précédentes, dans lequel la composition d'aérosol antiperspirant (AP) est anhydre.

10. Produit d'aérosol antiperspirant selon la revendication 10, dans lequel la composition est une suspension d'actif AP dans un fluide porteur.

11. Produit d'aérosol antiperspirant selon la revendication 10, dans lequel la composition comprend un agent de suspension.

12. Procédé de réalisation d'un bénéfice antiperspirant sur la peau humaine comprenant l'utilisation d'un produit d'aérosol antiperspirant selon l'une quelconque des revendications précédentes.

13. Procédé de réduction d'une détérioration corrosive sur une vanne d'aérosol et une coupelle de vanne associée d'un distributeur d'aérosol, la vanne d'aérosol comprenant un logement contenant la tige de vanne et un ressort, et étant contenue dans un corps de récipient par la coupelle de vanne, la détérioration corrosive étant occasionnée par une composition d'aérosol AP comprenant un propulseur volatile et un actif AP comprenant un composé de chlorure d'aluminium basique de formule Al₂OH_{4,4}Cl_{1,6} à Al₂OH_{4,9}Cl_{1,1}, et la réduction de détérioration corrosive étant réalisée par l'utilisation d'un revêtement protecteur sur la surface interne de la coupelle de vanne et l'addition de chlorure de calcium et glycine à l'actif AP de sorte que l'actif AP résultant présente un rapport molaire de calcium à aluminium supérieur à 1 : 20 et un rapport molaire de glycine à aluminium supérieur à 1 : 5.
